# EUROPEAN PATENT APPLICATION

(11) **EP 4 459 260 A1**
(43) Date of publication of application: **06.11.2024**
(21) Application number: 21969808.1
(22) Date of filing: 31.12.2021
(51) Int. Cl.: G01N 15/14, G01N 33/49

(54) **SAMPLE ANALYSIS DEVICE AND SAMPLE ANALYSIS METHOD**

(71) Applicant: Shenzhen Mindray Animal Medical Technology Co., Ltd., Shenzhen, Guangdong 518110 (CN)
(72) Inventor: KONG, Fangang, Shenzhen, Guangdong 518110 (CN); ZHANG, Xinjun, Shenzhen, Guangdong 518110 (CN); YANG, Zhuxiang, Shenzhen, Guangdong 518110 (CN); WANG, Shengxi, Shenzhen, Guangdong 518110 (CN)
(74) Representative: KIPA AB
(86) International application number: PCT/CN2021/143936
(87) International publication number: WO 2023/123463

(57) **Abstract**

A sample analysis apparatus and a sample analysis method. A specimen is prepared by means of a sample and a reagent, the sample being a blood sample or a body fluid sample; the specimen is tested to obtain one or more testing parameters of the sample with respect to particles; and for at least one testing parameter, control is performed according to the magnitude of the testing parameter to display the testing parameter with different precisions. By performing control according to the magnitude of a testing parameter to display the testing parameter with different precisions, a result requirement of a low-value sample such as a body fluid sample can be satisfied.

## Description

### TECHNICAL FIELD

The disclosure relates to a sample analysis apparatus and a sample analysis method.

### BACKGROUND

Some types of sample analysis apparatuses, such as cell analyzers, are capable of determining particles in samples such as blood samples and body fluid samples. Generally, there are few cell particles in the body fluid, but when a disease is developed or lesions occur in the related organs, relevant cell particles can be found in the body fluid.

At present, some sample analysis apparatuses are only capable of determining blood samples, but are incapable of determining body fluid samples. Other sample analysis apparatuses capable of analyzing and determining both blood samples and body fluid samples all provide two determination modes, namely, a blood determination mode and a body fluid determination mode. When the sample is a blood sample, it is necessary to switch the sample analysis apparatus to the blood determination mode, so that the sample is determined using relevant determination parameters in the blood determination mode to obtain a test result. When the sample is a body fluid sample, it is necessary to switch the sample analysis apparatus to the body fluid determination mode, so that the sample is determined using relevant determination parameters in the body fluid determination mode to obtain a test result.

### SUMMARY

The disclosure mainly provides a sample analysis apparatus and a sample analysis method, which will be described in detail below.

According to a first aspect, an embodiment provides a sample analysis apparatus, including:
a sample supply portion configured to supply a sample, the sample including a blood sample and a body fluid sample;
a reagent supply portion configured to supply a reagent;
a reaction portion configured to provide a reaction place for the sample and the reagent so as to prepare a test sample formed by reaction of the sample and the reagent;
a determination portion configured to test the test sample so as to obtain test data;
a processor that obtains one or more testing parameters of the sample with respect to particles according to the test data; and at least one testing parameter, the processor performing control according to the magnitude of the testing parameter to display the testing parameter with different precisions.

In an embodiment, the processor performing control according to the magnitude of the testing parameter to display the testing parameter with different precisions includes:
when the testing parameter is less than a threshold, the processor performs control to display the testing parameter with a first precision; conversely, the processor performs control to display the testing parameter with a second precision; where the first precision is higher than the second precision.

In an embodiment, the processor performs control according to the magnitude of a white blood cell count parameter to display the white blood cell count parameter with different precisions.

In an embodiment, the first precision is 0.001 * 10⁹/L, and the second precision is 0.01 * 10⁹/L.

In an embodiment, the processor performs control according to the magnitude of a red blood cell count parameter to display the red blood cell count parameter with different precisions.

In an embodiment, the first precision is 0.001 * 10¹²/L, and the second precision is 0.01 * 10¹²/L.

In an embodiment, in response to a setting command, the processor sets the threshold.

In an embodiment, the processor further generates an impact prompt for the same testing parameter as the sample currently tested of a next sample according to the magnitude of at least one testing parameter of the sample current.

In an embodiment, the impact prompt includes a quantitative value to indicate that when an actual value of the same testing parameter as the sample currently tested of the next sample is less than the quantitative value, it is required to perform blank counting first when the next sample is tested, otherwise, a resulting test value of the same testing parameter is unreliable.

In an embodiment, the processor generates an impact prompt for the white blood cell count parameter of the next sample according to the magnitude of the white blood cell count parameter of the current sample.

In an embodiment, if the white blood cell parameter of the current sample is greater than or equal to 60 * 10⁹/L and less than or equal to 100 * 10⁹/L, the quantitative value is 0.003 * 10⁹/L; if the white blood cell parameter of the current sample is greater than 100 * 10⁹/L and less than or equal to 200 * 10⁹/L, the quantitative value is 0.008 * 10⁹/L; if the white blood cell parameter of the current sample is greater than 200 * 10⁹/L and less than or equal to 300 * 10⁹/L, the quantitative value is 0.010 * 10⁹/L; and if the white blood cell parameter of the current sample is greater than 300 * 10⁹/L, the quantitative value is 0.015 * 10⁹/L.

In an embodiment, the processor generates an impact prompt for the red blood cell count parameter of the next sample according to the magnitude of the red blood cell count parameter of the current sample.

In an embodiment, if the red blood cell parameter of the current sample is greater than or equal to 12 * 10¹²/L and less than or equal to 17 * 10¹²/L, the quantitative value is 0.006 * 10¹²/L; if the red blood cell parameter of the current sample is greater than 17 * 10¹²/L and less than or equal to 20 * 10¹²/L, the quantitative value is 0.008 * 10¹²/L; if the red blood cell parameter of the current sample is greater than 20 * 10¹²/L and less than or equal to 25 * 10¹²/L, the quantitative value is 0.010 * 10¹²/L; and if the red blood cell parameter of the current sample is greater than 25 * 10¹²/L, the quantitative value is 0.013 * 10¹²/L.

In an embodiment, the testing parameter for which the impact prompt is generated, and the testing parameter displayed with different precisions under the control are the same testing parameter.

In an embodiment, the body fluid sample includes at least one of cerebrospinal fluid, pleural effusion, peritoneal effusion, pericardial effusion, and joint effusion.

According to a second aspect, an embodiment provides a sample analysis apparatus, including:
a sample supply portion configured to supply a sample, the sample including a blood sample and a body fluid sample;
a reagent supply portion configured to supply a reagent;
a reaction portion configured to provide a reaction place for the sample and the reagent so as to prepare a test sample formed by reaction of the sample and the reagent;
a determination portion configured to test the test sample so as to obtain test data; and
a processor configured to obtain one or more testing parameters of the sample with respect to particles according to the test data, the processor further generating an impact prompt for the same testing parameter as the sample currently tested of a next sample according to the magnitude of at least one testing parameter of the sample currently tested.

In an embodiment, the impact prompt includes a quantitative value to indicate that when an actual value of the same testing parameter as the sample currently tested of the next sample is less than the quantitative value, it is required to perform blank counting first when the next sample is tested, otherwise, a resulting test value of the same testing parameter is unreliable.

In an embodiment, the processor generates an impact prompt for the white blood cell count parameter of the next sample according to the magnitude of the white blood cell count parameter of the current sample.

In an embodiment, if the white blood cell parameter of the current sample is greater than or equal to 60 * 10⁹/L and less than or equal to 100 * 10⁹/L, the quantitative value is 0.003 * 10⁹/L; if the white blood cell parameter of the current sample is greater than 100 * 10⁹/L and less than or equal to 200 * 10⁹/L, the quantitative value is 0.008 * 10⁹/L; if the white blood cell parameter of the current sample is greater than 200 * 10⁹/L and less than or equal to 300 * 10⁹/L, the quantitative value is 0.010 * 10⁹/L; and if the white blood cell parameter of the current sample is greater than 300 * 10⁹/L, the quantitative value is 0.015 * 10⁹/L.

In an embodiment, the processor generates an impact prompt for the red blood cell count parameter of the next sample according to the magnitude of the red blood cell count parameter of the current sample.

In an embodiment, if the red blood cell parameter of the current sample is greater than or equal to 12 * 10¹²/L and less than or equal to 17 * 10¹²/L, the quantitative value is 0.006 * 10¹²/L; if the red blood cell parameter of the current sample is greater than 17 * 10¹²/L and less than or equal to 20 * 10¹²/L, the quantitative value is 0.008 * 10¹²/L; if the red blood cell parameter of the current sample is greater than 20 * 10¹²/L and less than or equal to 25 * 10¹²/L, the quantitative value is 0.010 * 10¹²/L; and if the red blood cell parameter of the current sample is greater than 25 * 10¹²/L, the quantitative value is 0.013 * 10¹²/L.

In an embodiment, the sample analysis apparatus further includes a single-sample mode; and in the single-sample mode, the processor generates an impact prompt for the same testing parameter as the sample currently tested of the next sample according to the magnitude of at least one testing parameter of the sample currently tested.

In an embodiment, for at least one testing parameter, the processor performs control according to the magnitude of the testing parameter to display the testing parameter with different precisions.

In an embodiment, the processor performing control according to the magnitude of the testing parameter to display the testing parameter with different precisions includes:
when the testing parameter is less than a threshold, the processor performs control to display the testing parameter with a first precision; conversely, the processor performs control to display the testing parameter with a second precision; where the first precision is higher than the second precision.

In an embodiment, the processor performs control according to the magnitude of a white blood cell count parameter to display the white blood cell count parameter with different precisions.

In an embodiment, the first precision is 0.001 * 10⁹/L, and the second precision is 0.01 * 10⁹/L.

In an embodiment, the processor performs control according to the magnitude of a red blood cell count parameter to display the red blood cell count parameter with different precisions.

In an embodiment, the first precision is 0.001 * 10¹²/L, and the second precision is 0.01 * 10¹²/L.

In an embodiment, in response to a setting command, the processor sets the threshold.

In an embodiment, the testing parameter for which the impact prompt is generated, and the testing parameter displayed with different precisions under the control are the same testing parameter.

In an embodiment, the body fluid sample includes at least one of cerebrospinal fluid, pleural effusion, peritoneal effusion, pericardial effusion, and joint effusion.

According to a third aspect, an embodiment provides a sample analysis method, including:
preparing a test sample from a sample and a reagent, the sample being a blood sample or a body fluid sample;
testing the test sample to obtain one or more testing parameters of the sample with respect to particles; and
for at least one testing parameter, performing control according to the magnitude of the testing parameter to display the testing parameter with different precisions.

In an embodiment, performing control according to the magnitude of the testing parameter to display the testing parameter with different precisions includes:
when the testing parameter is less than a threshold, performing control to display the testing parameter with a first precision; conversely, performing control to display the testing parameter with a second precision; where the first precision is higher than the second precision.

In an embodiment, performing control according to the magnitude of the testing parameter to display the testing parameter with different precisions includes:
performing control according to the magnitude of a white blood cell count parameter to display the white blood cell count parameter with different precisions; and/or
performing control according to the magnitude of a red blood cell count parameter to display the red blood cell count parameter with different precisions.

In an embodiment, the sample analysis method further includes: generating an impact prompt for the same testing parameter as the sample currently tested of a next sample according to the magnitude of at least one testing parameter of the sample currently tested.

In an embodiment, the impact prompt includes a quantitative value to indicate that when an actual value of the same testing parameter as the sample currently tested of the next sample is less than the quantitative value, it is required to perform blank counting first when the next sample is tested, otherwise, a resulting test value of the same testing parameter is unreliable.

In an embodiment, generating an impact prompt for the same testing parameter as the sample currently tested of a next sample according to the magnitude of at least one testing parameter of the sample currently tested includes:
generating an impact prompt for the white blood cell count parameter of the next sample according to the magnitude of the white blood cell count parameter of the current sample; and/or
generating an impact prompt for the red blood cell count parameter of the next sample according to the magnitude of the red blood cell count parameter of the current sample.

According to a fourth aspect, an embodiment provides a sample analysis method, including:
preparing a test sample from a sample and a reagent, the sample being a blood sample or a body fluid sample;
testing the test sample to obtain one or more testing parameters of the sample with respect to cells; and
generating an impact prompt for the same testing parameter as the sample currently tested of a next sample according to the magnitude of at least one testing parameter of the sample currently tested.

The impact prompt includes a quantitative value to indicate that when an actual value of the same testing parameter as the sample currently tested of the next sample is less than the quantitative value, it is required to perform blank counting first when the next sample is tested, otherwise, a resulting test value of the same testing parameter is unreliable.

In an embodiment, generating an impact prompt for the same testing parameter as the sample currently tested of a next sample according to the magnitude of at least one testing parameter of the current sample includes:
generating an impact prompt for the white blood cell count parameter of the next sample according to the magnitude of the white blood cell count parameter of the current sample; and/or
generating an impact prompt for the red blood cell count parameter of the next sample according to the magnitude of the red blood cell count parameter of the current sample.

In an embodiment, the sample analysis method further includes: for at least one testing parameter, performing control according to the magnitude of the testing parameter to display the testing parameter with different precisions.

In an embodiment, performing control according to the magnitude of the testing parameter to display the testing parameter with different precisions includes:
when the testing parameter is less than a threshold, performing control to display the testing parameter with a first precision; conversely, performing control to display the testing parameter with a second precision; where the first precision is higher than the second precision.

In an embodiment, performing control according to the magnitude of the testing parameter to display the testing parameter with different precisions includes:
performing control according to the magnitude of a white blood cell count parameter to display the white blood cell count parameter with different precisions; and/or
performing control according to the magnitude of a red blood cell count parameter to display the red blood cell count parameter with different precisions.

By means of the sample analysis apparatus and the sample analysis method of the above-mentioned embodiments, the impact prompt for the same testing parameter as the sample currently tested of the next sample is generated according to the magnitude of the at least one testing parameter of the current sample, so that the impact of the current sample on the next sample can be reduced, thereby assisting a user to ensure the accuracy of a test result of the next sample, such as a low-value sample.

### BRIEF DESCRIPTION OF THE DRAWINGS

FIG. 1 is a schematic structural diagram of a sample analysis apparatus according to an embodiment;
FIG. 2 is a schematic structural diagram of a sample analysis apparatus according to an embodiment;
FIG. 3 is a schematic structural diagram of an optical detection portion according to an embodiment;
FIG. 4 is a schematic structural diagram of an optical detection portion according to an embodiment;
FIG. 5 is a schematic structural diagram of an optical detection portion according to an embodiment;
FIG. 6 is a schematic structural diagram of an impedance method-based counting component according to an embodiment;
FIG. 7 is a schematic diagram of a result display interface according to an embodiment;
FIG. 8 is a schematic diagram of a result display interface according to an embodiment;
FIG. 9 is a partial schematic diagram of a result display interface according to an embodiment;
FIG. 10 is a schematic diagram of a result display interface according to an embodiment;
FIG. 11 is a partial schematic diagram of a result display interface according to an embodiment;
FIG. 12 is a partial schematic diagram of a result display interface according to an embodiment;
FIG. 13 is a schematic diagram of setting a white blood cell count threshold and a red blood cell count threshold according to an embodiment;
FIG. 14 is a flowchart of a sample analysis method according to an embodiment; and
FIG. 15 is a flowchart of a sample analysis method according to an embodiment.

### DESCRIPTION OF THE PREFERRED EMBODIMENTS

The disclosure will be further described in detail below through specific implementations in conjunction with the accompanying drawings. Associated similar element reference numerals are used for similar elements in different implementations. In the following implementations, many details are described such that the disclosure may be better understood. However, it may be effortlessly appreciated by persons skilled in the art that some of the features may be omitted, or may be substituted by other elements, materials, and methods in different cases. In certain cases, some operations involved in the disclosure are not displayed or described in the specification, which is to prevent a core part of the disclosure from being obscured by too much description. Moreover, for persons skilled in the art, the detailed description of the involved operations is not necessary, and the involved operations can be thoroughly understood according to the description in the specification and general technical knowledge in the art.

In addition, the characteristics, operations, or features described in the specification may be combined in any appropriate manner to form various implementations. In addition, the steps or actions in the method description may also be exchanged or adjusted in order in a way that is obvious to persons skilled in the art. Therefore, the various orders in the specification and the accompanying drawings are merely for the purpose of clear description of a certain embodiment and are not meant to be a necessary order unless it is otherwise stated that a certain order must be followed.

The serial numbers themselves for the components herein, for example, "first" and "second", are merely used to distinguish the described objects, and do not have any sequential or technical meaning. Moreover, as used in the disclosure, "connection" or "coupling", unless otherwise stated, includes both direct and indirect connections (couplings).

The sample analysis apparatus and sample analysis method of the disclosure can be applied to clinical diagnosis of human and/or animals. Blood samples according to some embodiments of the disclosure may be venous blood or arterial blood. Volumetric samples according to some embodiments of the disclosure includes at least one of cerebrospinal fluid, pleural effusion, peritoneal effusion, pericardial effusion, and joint effusion. The cerebrospinal fluid may be taken as an example to illustrate some characteristics of cell particles in a body fluid sample.

The cerebrospinal fluid (CSF) is a colorless and transparent fluid present in a ventricle and a subarachnoid space; 75% of the cerebrospinal fluid is present in the subarachnoid space, and the rest in a ventricular system. The cerebrospinal fluid mainly functions to protect the brain and the spinal cord from external concussive injuries, to regulate an intracranial pressure, to provide a central nervous system with nutrients and to carry their metabolites out, and to participate in neuroendocrine regulation, etc. When there are symptoms of meningeal irritation, for example, a meningeal infectious disease, suspected intracranial hemorrhage such as subarachnoid hemorrhage, central nervous system neoplasms, and unexplained severe headache, coma, convulsion or paralysis, it is necessary to determine the cerebrospinal fluid, for example, count and classify white blood cells thereof, and count red blood cells, etc.

Taking an animal as an example, in normal conditions, a normal value of the white blood cells in the cerebrospinal fluid of the animal shall be less than 10 * 10⁹/L, and a specific value is related to the type of the animal, while the red blood cells cannot be detected in the normal conditions. According to some diagnostic criteria, when the white blood cells in the cerebrospinal fluid are 10 * 10⁹/L to 50 * 10⁹/L, it is indicated that the white blood cells slightly increase; when the white blood cells in the cerebrospinal fluid are 50 * 10⁹/L to 100 * 10⁹/L, it is indicated that the white blood cells moderately increase; and when the white blood cells in the cerebrospinal fluid are greater than 200 * 10⁹/L, it is indicated that the white blood cells significantly increase.

With regard to purulent meningitis, the white blood cells in the cerebrospinal fluid significantly increase, generally greater than 1,000 * 10⁹/L, and neutrophil granulocytes are predominant.

With regard to tuberculous meningitis, the white blood cells in the cerebrospinal fluid moderately increase, generally not more than 500 * 10⁹/L, and neutrophil granulocytes are predominant at an early stage of the disease; and several days later, neutrophil granulocytes decrease rapidly while lymphocytes increase, resulting in that the lymphocytes are predominant.

With regard to viral encephalitis and meningitis, the white blood cells in the cerebrospinal fluid slightly increase, and the lymphocytes are predominant.

With regard to cryptococcus neoformans meningitis, the white blood cells in the cerebrospinal fluid moderately increase, and the lymphocytes are predominant.

The subarachnoid hemorrhage, intraventricular and subarachnoid hemorrhage manifest with a uniform bloody cerebrospinal fluid at an early stage, with massive red blood cells and an obvious increase in neutrophil granulocytes.

With regard to the central nervous system neoplasms, the number of cells in the cerebrospinal fluid are normal or slightly high, and the lymphocytes are predominant.

With regard to a cerebral parasitic disease, the number of cells in the cerebrospinal fluid may increase, and eosinophil granulocytes may increase, probably greater than 60%.

Therefore, in some cases, on the basis of the clinical characteristics of the cerebrospinal fluid, an instrument is required to be capable of performing classified testing on white blood cells, namely to have the ability to distinguish neutrophil granulocytes, lymphocytes and eosinophil granulocytes; and normally, the white blood cells in the cerebrospinal fluid are less than 10 * 10⁶/L, and the instrument is required to give a test result in a test range of 0 to 10 * 10⁶/L. Normally, it is required that no red blood cells are found in the cerebrospinal fluid, and the instrument is required to give an accurate test result in a test range of 0 to 10 * 10⁹/L.

The inventor found that when a sample analysis apparatus currently available in the market tests a blood sample in a blood determination mode, the number of white blood cells is mostly given with two decimal points (i.e. the precision is 0.01 * 10⁹/L), and some instruments even provide numbers with one decimal point (i.e. the precision is 0.1 * 10⁹/L); while the number of red blood cells is mostly given with two decimal points (i.e. the precision is 0.01 * 10¹²/L); and when testing a low-value sample such as a body fluid sample, the sample analysis apparatus cannot give an accurate test result of the low-value sample.

The inventor also found that particle test items (or cell test items) were significantly different when the sample analysis apparatus determines a blood sample and a body fluid sample, for example, taking the sample analysis apparatus of Mindray BC-6900 model as an example, for the blood sample, there are 36 report parameters, 18 study parameters, 2 histograms, 6 scatter diagrams and 8 measurement modes; and for the body fluid sample, provided are 7 report parameters, 7 study parameters, 1 histogram, 1 scatter diagram, and one measurement mode. The measurement modes herein mainly refer to a set of particle test items, which will be described in detail below.

For example, there are 8 measurement modes for a blood sample: CBC + DIFF (abbreviated as a CD mode), CBC, CBC + DIFF + RET (abbreviated as a CDR mode), CBC + RET (abbreviated as a CR mode), CBC + NRBC (abbreviated as a CN mode), CBC + DIFF + NRBC (abbreviated as a CDN mode), CBC + DIFF + RET + NRBC (abbreviated as a CDRN mode), and RET.

For example, there is 1 measurement mode for a body fluid sample: CBC + DIFF (abbreviated as a CD mode).

As described above, CBC typically includes white blood cell counting, red blood cell counting by using an impedance method, and platelet counting by using an impedance method; DIFF typically includes white blood cell classification; RET typically includes detecting platelets, reticulocytes, and immature platelets; and NRBC typically includes counting nucleated red blood cells.

It can be seen that the measurement mode or the set of particle test items of the body fluid sample is one of the measurement modes or the sets of particle test items of the blood sample.

As described above, in the prior art, when a blood sample is tested, it is required to select a blood determination mode for the blood sample, and display, for example, 36 report parameters, 18 study parameters, 2 histograms, and 6 scatter diagrams, on a result display interface; and when a body fluid sample is tested, it is required to select a body fluid determination mode for the body fluid sample, and display, for example, 7 report parameters, 7 study parameters, 1 histogram, and 1 scatter diagram, on the result display interface.

In some embodiments, the disclosure provides a sample analysis apparatus and a sample analysis method, which do not distinguish a blood determination mode from a body fluid determination mode, but allow a blood sample and a body fluid sample to be analyzed and determined in the same determination mode. For example, the blood determination mode is used uniformly, or the sample analysis apparatus has only one determination mode, i.e. the blood determination mode; and the display is then performed with different precisions according to the magnitude of test results. For example, the display is performed with the higher precisions at low values and with the lower precisions at high values. According to an example, it can be that at low values, a white blood cell count and a red blood cell count are displayed as 3 significant digits, that is, the precisions are 0.001 * 10⁹/L and 0.001 * 10¹²/L respectively; at high values, the white blood cell count and the red blood cell count are displayed as 2 significant digits, that is, the precisions are 0.01 * 10⁹/L and 0.01 * 10¹²/L respectively; for example, when the white blood cell count is less than 0.50 * 10⁹/L, a result is displayed as 3 significant digits, that is, the precision is 0.001 * 10⁹/L; conversely, the result is displayed as 2 significant digits, that is, the precision is 0.01 * 10⁹/L; when the red blood cell count is less than 0.50 * 10¹²/L, a result is displayed as 3 significant digits, that is, the precision is 0.001 * 10¹²/L; conversely, the result is displayed as 2 significant digits, that is, the precision is 0.01 * 10¹²/L; and several specific examples will be shown in the following table.

| The number of significant digits | WBC (10⁹/L) | RBC (10¹²/L) |
|---|---|---|
| 3 | 0.023 | 0.125 |
| 3 | 0.323 | 0.258 |
| 2 | 8.72 | 5.64 |
| 2 | 9.83 | 6.98 |

In this case, the decimal point positions of 95% or above of test results are consistent with those displayed in test results of blood sample analysis in the prior art (that is, the precisions are consistent). Only the low-value counting results of white blood cells and red blood cells of some samples are 3 significant digits, which do not affect the user's interpretation of normal blood cell analysis reports.

Therefore, when the test results are displayed, the blood sample and the body fluid sample can use the same display interface, that is, the same parameters can be displayed; for example, the parameters required for the blood sample are displayed by uniformly using a result display interface of the blood sample, but some parameters are displayed with different precisions due to the different magnitudes of the parameters.

The display problem is described above, and the problem of impact of previous and later samples will be described below.

The body fluid sample, such as the cerebrospinal fluid, requires an accurate test value for a white blood cell test result of less than 0.001 * 10⁹/L to 0.01 * 10⁹/L and an accurate test value for a red blood cell test result of less than 0.001 * 10¹²/L to 0.01 * 10¹²/L. This requires that a high-value sample, such as a blood sample, cannot be tested prior to testing a body fluid sample because carryover may affect the accuracy of a test result of the next low-value sample when the high-value sample is tested. Considering the impact of the carryover on the count result of the low-value sample, after the sample is tested, it is prompted that the test result of the next sample may be impacted; if the next sample to be tested by the user is a normal blood sample, the impact is neglectable; if the next sample to be tested is a body fluid sample, or a low-value blood sample, and if it is prompted that an impacted value affects the clinical diagnosis of the test sample, the user needs to perform a blank sample (no sample being aspirated) test to ensure the accuracy of the test results of the low-value blood sample and the body fluid sample. With such a solution, it is possible to achieve accurate testing of human and animal samples, such as the cerebrospinal fluid, the pleural effusion, the peritoneal effusion, the pericardial effusion, and the joint effusion. Therefore, in some embodiments of the disclosure, this problem is solved by giving the impact of the current sample on the next sample, especially a quantitative impact. When there is an impact, one or more blank tests or blank counts (i.e. the determination without sample aspiration) can be interposed in the testing process, such that the impact of the current sample on the next sample can be greatly reduced, and if the current sample is a high-value sample such as a blood sample, the determination of the next sample, which is a low-value sample such as a body fluid sample, is also not affected. In some examples, by interposing the blank counts in the testing process, a blank counting result of white blood cells can be less than 0.002 * 10⁹/L, and a blank count of red blood cell passages can be less than 0.002 * 10⁹/L.

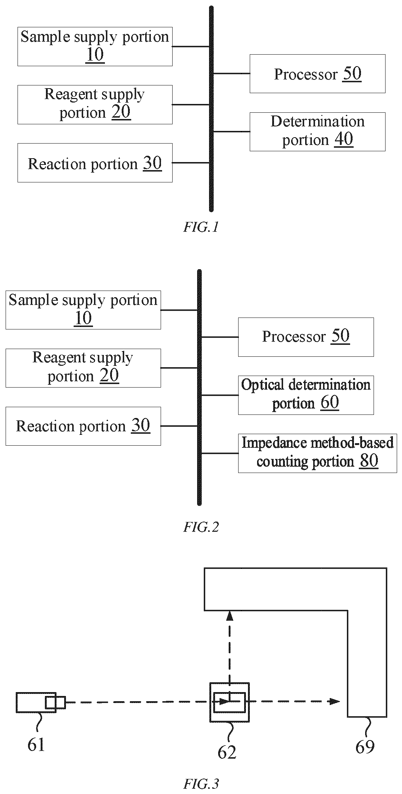

Referring to FIG. 1, a sample analysis apparatus according to some embodiments of the disclosure includes a sample supply portion 10, a reagent supply portion 20, a reaction portion 30, a determination portion 40, and a processor 50. In some specific embodiments, the sample supply portion 10 is configured to supply a sample; the sample may be a blood sample or a body fluid sample; the body fluid sample may be, for example, cerebrospinal fluid, pleural effusion, peritoneal effusion, pericardial effusion, joint effusion, etc.; the reagent supply portion 20 is configured to supply a reagent, such as a hemolytic agent, a fluorescent agent and/or a diluent, etc.; the reaction portion 30 is configured to provide a reaction place for the sample and the reagent so as to prepare a test sample formed by reaction of the sample and the reagent; the determination portion 40 is configured to detect the prepared test sample, or to detect the test sample so as to obtain test data; and the processor 50 is configured to calculate a test result according to the test data, for example, one or more testing parameters of the sample with respect to particles according to the test data. The processor 50 according to some embodiments of the disclosure includes, but is not limited to, a central processing unit (CPU), a micro controller unit (MCU), a field-programmable gate array (FPGA), a digital signal processor (DSP) and other apparatuses for interpreting computer instructions and processing data in computer software. In some embodiments, the processor 50 is configured to execute each computer application program in a non-transitory computer-readable storage medium, such that the sample analysis apparatus executes a corresponding test procedure.

The components are further described below.

In some embodiments, the sample supply portion 10 may include a sample needle which performs a two-dimensional or three-dimensional movement in space by means of a two-dimensional or three-dimensional driving mechanism, such that the sample needle may move to aspirate a sample in a container (such as a sample tube) carrying the sample, then move to, for example, the reaction portion 30, which is configured to provide a reaction place for the tested sample and the tested reagent, and add the sample to the reaction portion 30.

In some embodiments, the reagent supply portion 20 may include a region for carrying a reagent container and a reagent liquid path communicating the reagent container with the reaction portion 30, where a reagent is added from the reagent container to the reaction portion 30 through the reagent liquid path. In some embodiments, the reagent supply portion 20 may further include a reagent needle which performs a two-dimensional or three-dimensional movement in space by means of a two-dimensional or three-dimensional driving mechanism, such that the reagent needle may move to aspirate a reagent in a reagent container, then move to, for example, the reaction portion 30, which is configured to provide a reaction place for the tested sample and the tested reagent, and add the reagent to the reaction portion 30.

The reaction portion 30 may include one or more reaction cells. The reaction portion 30 is configured to provide a treatment place or a reaction place for the sample and the reagent. The same reaction cell may be shared for different particle test items; and different reaction cells may also be used for different particle test items.

A test sample to be tested may be obtained by treating the sample with the reagent. In some embodiments, the reagents include one or more of a hemolytic agent, a fluorescent agent, and a diluent. The hemolytic agent is a reagent capable of hemolyzing red blood cells in a blood sample and a body fluid sample. Specifically, the hemolytic agent may be any one or a combination of a cationic surfactant, a non-ionic surfactant, an anionic surfactant and an amphiphilic surfactant. The fluorescent agent is configured to stain blood cells, and the specific type is selected depending on the test entries. An isotonic electrolyte diluent may be configured to maintain the morphology of cell particles so as to prepare a test sample for counting with an impedance method, etc.

In some embodiments, referring to FIG. 2, the determination portion 40 includes an optical detection portion 60 and/or an impedance method-based counting component 80, which will be specifically described below.

In some embodiments, the determination portion 40 may include the optical detection portion 60, and the optical detection portion 60 is capable of detecting a sample by using the laser scattering principle. The principle thereof is as follows: cells are irradiated by laser light, and the cells are classified and counted by collecting optical signals generated after the cells are irradiated, such as scattered light and fluorescence. Of course, in some embodiments, if the cells are not treated with the fluorescent agent, no fluorescence can be certainly collected. The optical detection portion 60 in the determination portion 40 will be described below.

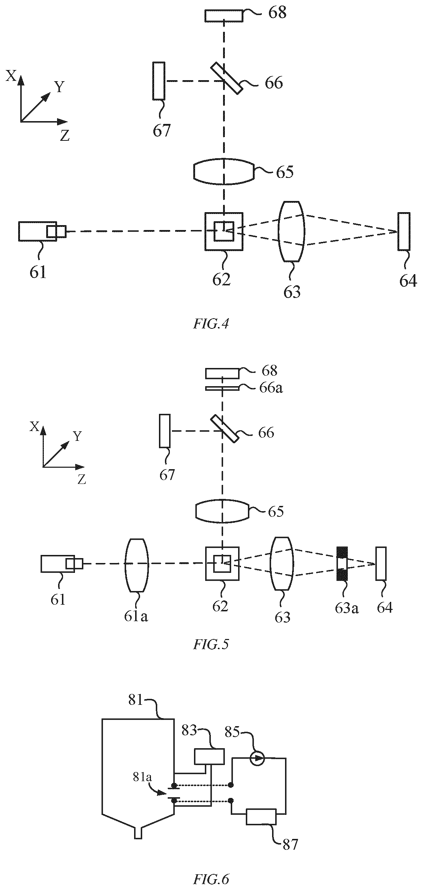

Referring to FIG. 3, the optical detection portion 60 may include a light source 61, a flow cell 62, and an optical detector 69. The flow cell 62 is in communication with the reaction portion 30 and is configured to allow cells of the test sample to be tested to pass through one by one; and the light source 61 is configured to irradiate the cells passing through the flow cell 62, and the optical detector 69 is configured to obtain an optical signal of the cells passing through the flow cell 62. FIG. 4 shows a specific example of the optical detection portion 60. The optical detector 69 may include a lens group 63 for collecting forward-scattered light, a photoelectric detector 64 for converting the collected forward-scattered light from an optical signal to an electrical signal, a lens group 65 for collecting side-scattered light and side fluorescence, a dichroscope 66, a photoelectric detector 67 for converting the collected side-scattered light from an optical signal to an electrical signal, and a photoelectric detector 68 for converting the collected side fluorescence from an optical signal to an electrical signal; and the dichroscope 66 is configured to split light, and divides the mixed side-scattered light and side fluorescence into two paths, one path for the side-scattered light, and the other path for the side fluorescence. It should be noted that the optical signals herein may be either optical signals or electrical signals obtained by conversion from the optical signals, and the optical signals and electrical signals are essentially the same in information contained in representing cell detection results.

The structure of the optical detection portion 60 shown in FIG. 4 is used as an example to explain how the optical detection portion 60 specifically obtains an optical signal of the test sample to be tested.

The flow cell 62 is configured to allow the cells of the test sample to be tested to pass through one by one. For example, in the reaction portion 30, the red blood cells in the sample are hemolyzed by using some reagents such as the hemolytic agent, or are further stained by using the fluorescent agent, and then the cells in the prepared test sample to be tested successively pass through the flow cell 62 one by one by using a sheath flow technology. The Y-axis direction in the figure is the direction of movement of the cells in the test sample to be tested, and it should be noted that the Y-axis direction in the figure is a direction perpendicular to a paper plane. The light source 61 is configured to irradiate the cells passing through the flow cell 62. In some embodiments, the light source 61 is a laser, such as a helium-neon laser or a semiconductor laser. The light from the light source 61 will be scattered all around when irradiating the cells in the flow cell 62. Therefore, when the cells in the prepared test sample to be tested pass through the flow cell 62 one by one by virtue of a sheath flow, the light emitted by the light source 61 irradiates the cells passing through the flow cell 62, and the light irradiated on the cells will be scattered all around. The forward-scattered light (for example, in a Z-axis direction in the figure) is collected by the lens group 63 to reach the photoelectric detector 64, such that an information processing portion 70 can obtain the forward-scattered light information of the cells from the photoelectric detector 64; and also, side light (for example, in an X-axis direction in the figure) is collected by the lens group 65 in a direction perpendicular to the light irradiated on the cells, and the collected side light is then reflected and refracted by the dichroscope 66. Side-scattered light in the side light is reflected when passing through the dichroscope 66, and then reaches the corresponding photoelectric detector 67, and side fluorescence in the side light is refracted or transmitted and then also reaches the corresponding photoelectric detector 68, such that the processor 50 can obtain side-scattered light information of the cells from the photoelectric detector 67 and obtain side fluorescence information of the cells from the photoelectric detector 68. Referring to FIG. 5, another example of the optical detection portion 60 is illustrated. To make the performance of the light from the light source 61 irradiated on the flow cell 62 better, a collimating lens 61a may be introduced between the light source 61 and the flow cell 62. The light emitted by the light source 61 is collimated by the collimating lens 61a and then irradiated on the cells passing through the flow cell 62. In some examples, in order to reduce noise in the collected fluorescence (i.e. no interference from other light), an optical filter 66a may be further provided in front of the photoelectric detector 68, and the side fluorescence after splitting by the dichroscope 66 will pass through the optical filter 66a before reaching the photoelectric detector 68. In some embodiments, after the lens group 63 collects the forward-scattered light, a diaphragm 63a is further introduced to limit an angle of the forward-scattered light that finally reaches the photoelectric detector 64, for example, the forward-scattered light is limited to be low-angle (or small-angle) forward-scattered light.

The white blood cells can be classified and counted by using a laser scattering method, and the optical detection portion 60 described above is construed as an example. The scattered light generated by irradiating the cells with a laser beam is related to the size of the cells, the refractive index of cell membranes and the refractive index of internal structures of the cells. According to a scattered light signal, a distribution diagram showing the size of blood cells and the internal information of the cells can be obtained, and is called a scattergram.

In some embodiments, referring to FIG. 6, the impedance method-based counting component 80 includes a counting cell 81, a pressure source 83, a constant-current power source 85, and a voltage pulse detection component 87. The counting cell 81 includes an aperture 81a, and the counting cell 81 is configured to receive the test sample from the reaction portion 30. The pressure source 83 is configured to provide a pressure such that the cells contained by the test sample in the counting cell 81 pass through the aperture 81a. Two ends of the constant-current power source 85 are electrically connected to two ends of the aperture 81a respectively. The voltage pulse detection component 87 is electrically connected to the constant-current power source 85, and is configured to detect voltage pulses generated when the cells pass through the aperture 81a.

The white blood cells can also be classified and counted by using an impedance method, and the above-described impedance method-based counting component 80 is construed as an example. The red blood cells, platelets, etc. can also be counted by using an impedance method, and the above-described impedance method-based counting component 80 is construed as an example. Specifically, by receiving voltage pulses associated with the cells, a histogram of the cells can be statistically formed so that classification and counting of the cells can be completed.

For the body fluid sample, what's the most concerned are the test results of white blood cells and red blood cells. In some embodiments, the blood sample and body fluid sample are tested in the same determination mode, that is, the blood sample and the body fluid sample are not distinguished on the sample analysis apparatus, test results of the low-value white blood cells and red blood cells are displayed with the high precisions, for example, displayed as 3 significant digits, and test results of white blood cells and red blood cells of other values are displayed with non-high precisions, for example, displayed as 2 significant digits.

As described above, the processor 50 obtains one or more testing parameters of the sample with respect to particles according to the test data. In some embodiments, for at least one testing parameter, the processor 50 performs control according to the magnitude of the testing parameter to display the testing parameter with different precisions; for example: when the testing parameter is less than a threshold, the processor 50 performs control to display the testing parameter with a first precision; conversely, when the testing parameter is greater than or equal to the threshold, the processor 50 performs control to display the testing parameter with a second precision; where the first precision is higher than the second precision. The threshold mentioned above may be a factory default setting, or may be set and modified by the user; for example, in response to a setting command, the processor 50 sets a threshold.

In some specific embodiments, the processor 50 performs control according to the magnitude of a white blood cell count parameter to display the white blood cell count parameter with different precisions. For example, when the white blood cell count parameter is less than a threshold, the processor 50 performs control to display the white blood cell count parameter with the first precision; conversely, when the white blood cell count parameter is greater than or equal to the threshold, the processor 50 performs control to display the white blood cell count parameter with the second precision. In some embodiments, the first precision is 0.001 * 10⁹/L, and the second precision is 0.01 * 10⁹/L.

In some specific embodiments, the processor 50 performs control according to the magnitude of a red blood cell count parameter to display the white blood cell count parameter with different precisions. For example, when the red blood cell count parameter is less than a threshold, the processor 50 performs control to display the red blood cell count parameter with the first precision; conversely, when the red blood cell count parameter is greater than or equal to the threshold, the processor 50 performs control to display the white blood cell count parameter with the second precision. In some embodiments, the first precision is 0.001 * 10¹²/L, and the second precision is 0.01 * 10¹²/L.

In some examples, if the white blood cell test result and the red blood cell test result may affect a next low-value sample test result after the current sample is tested, a quantitative prompt of the impact is given on an interface, which prompts the user to evaluate whether it is required to preform a blank test (a test performed without aspirating a blood sample) before the next sample is tested, and whether to clean a tubing in order to avoid the impact on a next sample test result.

In some embodiments, the processor 50 generates an impact prompt for the same testing parameter as the sample currently tested of the next sample according to the magnitude of at least one testing parameter of the sample currently tested. In some embodiments, the impact prompt includes a quantitative value to indicate that when an actual value of the same testing parameter as the sample currently tested of the next sample is less than the quantitative value, it is required to perform blank counting first when the next sample is tested, otherwise, a resulting test value of the same testing parameter is unreliable.

In some specific embodiments, the processor 50 generates an impact prompt for the white blood cell count parameter of the next sample according to the magnitude of the white blood cell count parameter of the current sample. For example, if the white blood cell parameter of the current sample is greater than or equal to 60 * 10⁹/L and less than or equal to 100 * 10⁹/L, the quantitative value is 0.003 * 10⁹/L; if the white blood cell parameter of the current sample is greater than 100 * 10⁹/L and less than or equal to 200 * 10⁹/L, the quantitative value is 0.008 * 10⁹/L; if the white blood cell parameter of the current sample is greater than 200 * 10⁹/L and less than or equal to 300 * 10⁹/L, the quantitative value is 0.010 * 10⁹/L; and if the white blood cell parameter of the current sample is greater than 300 * 10⁹/L, the quantitative value is 0.015 * 10⁹/L. It should be noted that specific quantitative values are related to the model and performance of the sample analysis apparatus itself, and each quantitative value may be obtained by means of actual measurement.

In some specific embodiments, the processor 50 generates an impact prompt for the red blood cell count parameter of the next sample according to the magnitude of the red blood cell count parameter of the current sample. For example, if the red blood cell parameter of the current sample is greater than or equal to 12 * 10¹²/L and less than or equal to 17 * 10¹²/L, the quantitative value is 0.006 * 10¹²/L; if the red blood cell parameter of the current sample is greater than 17 * 10¹²/L and less than or equal to 20 * 10¹²/L, the quantitative value is 0.008 * 10¹²/L; if the red blood cell parameter of the current sample is greater than 20 * 10¹²/L and less than or equal to 25 * 10¹²/L, the quantitative value is 0.010 * 10¹²/L; and if the red blood cell parameter of the current sample is greater than 25 * 10¹²/L, the quantitative value is 0.013 * 10¹²/L. It should be noted that specific quantitative values are related to the model and performance of the sample analysis apparatus itself, and each quantitative value may be obtained by means of actual measurement.

In some embodiments, the sample analysis apparatus includes a single-sample mode and/or a multi-sample mode. The single-sample mode, which means that the user manually puts a single sample into the sample analysis apparatus, is a means or mode of manual sample injection. The multi-sample mode, which means that the user puts a plurality of samples into the sample analysis apparatus in batches, is a means or mode of automatic sample injection. Considering that the number of body fluid samples is less than that of blood samples, in view of ease of sample injection, it is generally recommended that the user detects the body fluid samples by means of manual sample injection and detects the blood samples by means of automatic sample injection. In some embodiments, only in the single-sample mode or in a manual sample injection mode, the processor 50 gives the above-mentioned impact prompt, for example, generates an impact prompt for the same testing parameter as the sample currently tested of the next sample according to the magnitude of at least one testing parameter of the current sample; or, the processor generates an impact prompt for the same testing parameter as the sample currently tested of a sample currently to be determined in the single-sample mode, according to the magnitude of at least one testing parameter of the previous sample.

As described above, in some embodiments, for at least one testing parameter, the processor 50 performs control according to the magnitude of the testing parameter to display the testing parameter with different precisions; in some embodiments, the processor 50 generates an impact prompt for the same testing parameter as the sample currently tested of the next sample according to the magnitude of at least one testing parameter of the sample currently tested; and in some embodiments, the testing parameter for which the impact prompt is generated, and the testing parameter displayed with different precisions under the control are the same testing parameter, for example, both being the white blood cell count and/or the red blood cell count.

The processor 50 displays the testing parameter of the sample with respect to the particles by generating a result display interface.

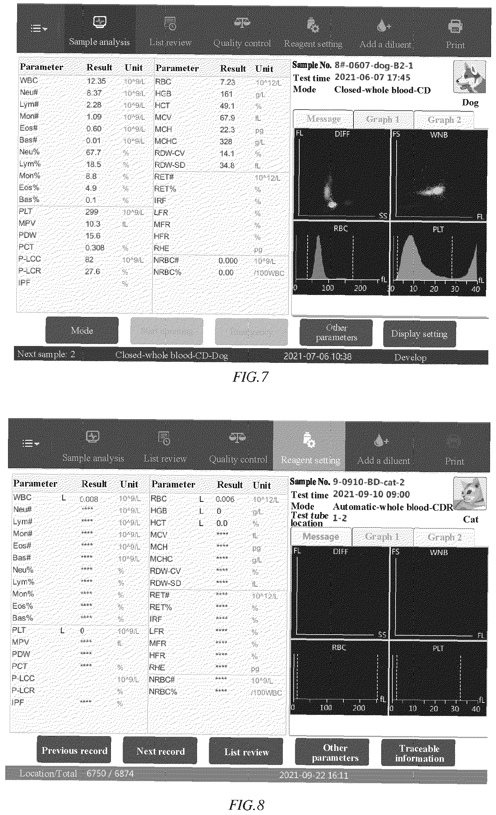

FIG. 7 shows an example, in which when the count of white blood cells (WBCs) is greater than or equal to 0.50 * 10⁹/L, the WBC testing parameter is displayed as 2 significant digits, i.e. the precision is 0.01 * 10⁹/L; and when the count of red blood cells (RBCs) is greater than or equal to 0.50 * 10¹²/L, the RBC testing parameter is displayed as 2 significant digits, i.e. the precision is 0.01 * 10¹²/L. FIG. 8 shows another example, in which when the count of white blood cells (WBCs) is less than 0.50 * 10⁹/L, the WBC testing parameter is displayed as 3 significant digits, i.e. the precision is 0.001 * 10⁹/L; and when the count of red blood cells (RBCs) is less than 0.50 * 10¹²/L, the RBC testing parameter is displayed as 3 significant digits, i.e. the precision is 0.001 * 10¹²/L.

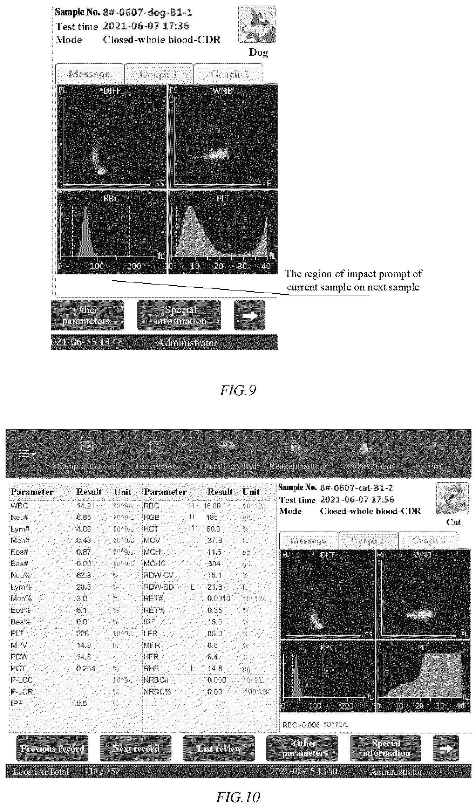

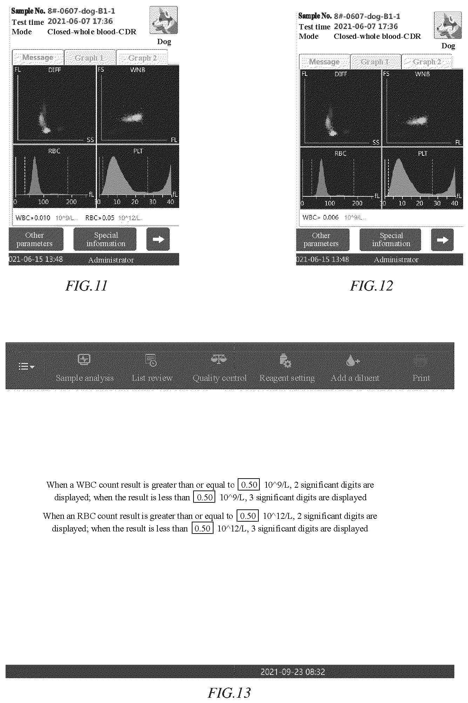

When the white blood cell count and/or the red blood cell count may impact the test result of the next sample, a possible impact value (i.e. the above quantitative value) on the next sample is prompted on the result display interface, and the impact value is related to the design of a sample analysis apparatus, and may be different somewhat depending on different models and be obtained by means of actual measurement. The prompt may be displayed in a specific region of the result display interface, for example, a region shown in FIG. 9, which serves as a prompt region. FIG. 10 shows an example that the RBC result of the current sample is 16.08 * 10¹²/L, which is slightly higher; there is a prompting content in the prompt region, which prompts that a next sample RBC test result may be impacted as 0.006 * 10¹²/L, i.e. a corresponding quantitative value is 0.006 * 10¹²/L; and if the next sample to be tested is the cerebrospinal fluid, the user needs to perform a blank counting before testing the cerebrospinal fluid sample to eliminate the impact of the current test sample on a cerebrospinal fluid sample test result. FIG. 11 shows another example (only a part of the result display interface is displayed), in which WBCs and RBCs both have prompting contents in the prompt regions, which prompt impact values with respect to WBC and RBC test results of the next test sample. What's shown in this figure are: the quantitative value of WBCs of the next test sample is 0.010 * 10⁹/L, and the quantitative value of RBCs of the next test sample is 0.05 * 10¹²/L; and the user needs to determine whether to perform a blank counting according to the type of the next sample. FIG. 12 shows another example (only a part of the result display interface is displayed), in which WBCs have a prompting content in the prompt region, which prompts an impact value with respect to the WBC test result of the next test sample. What's shown in this figure is: the quantitative value of the WBCs of the next test sample is 0.006 * 109/L; and the user needs to determine whether to perform a blank counting according to the type of the next sample.

If WBCs or RBCs have a display content in the prompt region during the test of the body fluid sample or the low-value blood sample, the user performs a blank sample (no sample aspirated) test to confirm that the current test sample does not affect the next sample result parameter. With the above solution, the user can be prompted to perform the blank sample (no sample aspirated) test when needed. When the sample results of WBCs and RBCs are lower than the corresponding thresholds, WBCs are displayed as 3 significant digits at the magnitude of 10⁹/L; RBCs are displayed as 3 significant digits at the magnitude of 10¹²/L; when the sample results of WBCs and RBCs are greater than or equal to the corresponding thresholds, WBCs are displayed as 2 significant digits at the magnitude of 10⁹/L; and RBCs are displayed as 2 significant digits at the magnitude of 10¹²/L.

In some embodiments, the interface may be provided to set the threshold of WBCs and/or the threshold of RBCs. For example, FIG. 13 shows an example. In this figure, the threshold of WBCs is set as 0.05 * 10⁹/L, and the threshold of RBCs is set as 0.05 * 10¹²/L.

In some examples, the low-value sample is displayed by increasing the multiples of decimal points, and the user is prompted to conform whether to perform blank counting before testing the next sample, so that analyzing the body fluid sample is completed in the same determination mode, and an accurate body fluid sample test result is obtained.

The disclosure further discloses a sample analysis method, which will be specifically described below.

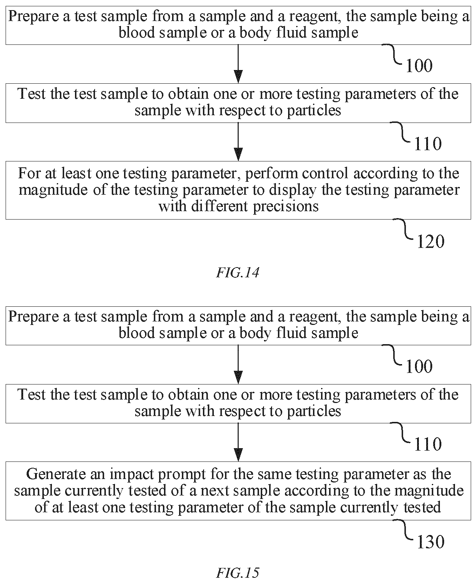

Referring to FIG. 14, the sample analysis method in some embodiments includes the following steps:
in step 100, preparing a test sample from a sample and a reagent, the sample being a blood sample or a body fluid sample;
in step 110, testing the test sample to obtain one or more testing parameters of the sample with respect to particles; and
in step 120, for at least one testing parameter, performing control according to the magnitude of the testing parameter to display the testing parameter with different precisions.

In some embodiments, when the testing parameter involved in step 120 is less than a threshold, step 120 includes performing control to display the testing parameter with a first precision; conversely, when the testing parameter is greater than or equal to the threshold, step 120 includes performing control to display the testing parameter with a second precision; where the first precision is higher than the second precision. The threshold mentioned above may be a factory default setting, or may be set and modified by the user; for example, in response to a setting command, step 120 includes setting the threshold.

In some embodiments, the testing parameter involved in step 120 includes a white blood cell count or white blood cell count parameter, and/or a red blood cell count or red blood cell count parameter.

Therefore, in some specific embodiments, step 120 includes performing control according to the magnitude of the white blood cell count parameter to display the white blood cell count parameter with different precisions. For example, when the white blood cell count parameter is less than the threshold, step 120 includes performing control to display the white blood cell count parameter with the first precision; conversely, when the white blood cell count parameter is greater than or equal to the threshold, step 120 includes performing control to display the white blood cell count parameter with the second precision. In some embodiments, the first precision is 0.001 * 10⁹/L, and the second precision is 0.01 * 10⁹/L.

In some specific embodiments, step 120 includes performing control according to the magnitude of the red blood cell count parameter to display the white blood cell count parameter with different precisions. For example, when the red blood cell count parameter is less than the threshold, step 120 includes performing control to display the red blood cell count parameter with the first precision; conversely, when the red blood cell count parameter is greater than or equal to the threshold, step 120 includes performing control to display the white blood cell count parameter with the second precision. In some embodiments, the first precision is 0.001 * 10¹²/L, and the second precision is 0.01 * 10¹²/L.

Referring to FIG. 15, the sample analysis method in some embodiments includes the following steps:
in step 100, preparing a test sample from a sample and a reagent, the sample being a blood sample or a body fluid sample;
in step 110, testing the test sample to obtain one or more testing parameters of the sample with respect to cells; and
in step 130, generating an impact prompt for the same testing parameter as the sample currently tested of a next sample according to the magnitude of at least one testing parameter of the sample currently tested.

In some embodiments, the impact prompt generated in step 130 includes a quantitative value to indicate that when an actual value of the same testing parameter as the sample currently tested of the next sample is less than the quantitative value, it is required to perform blank counting first when the next sample is tested, otherwise, a resulting test value of the same testing parameter is unreliable.

In some specific embodiments, step 130 includes generating an impact prompt for the white blood cell count parameter of the next sample according to the magnitude of the white blood cell count parameter of the current sample. For example, if the white blood cell parameter of the current sample is greater than or equal to 60 * 10⁹/L and less than or equal to 100 * 10⁹/L, the quantitative value is 0.003 * 10⁹/L; if the white blood cell parameter of the current sample is greater than 100 * 10⁹/L and less than or equal to 200 * 10⁹/L, the quantitative value is 0.008 * 10⁹/L; if the white blood cell parameter of the current sample is greater than 200 * 10⁹/L and less than or equal to 300 * 10⁹/L, the quantitative value is 0.010 * 10⁹/L; and if the white blood cell parameter of the current sample is greater than 300 * 10⁹/L, the quantitative value is 0.015 * 10⁹/L. It should be noted that specific quantitative values are related to the model and performance of the sample analysis apparatus itself, and each quantitative value may be obtained by means of actual measurement.

In some specific embodiments, step 130 includes generating an impact prompt for the red blood cell count parameter of the next sample according to the magnitude of the red blood cell count parameter of the current sample. For example, if the red blood cell parameter of the current sample is greater than or equal to 12 * 10¹²/L and less than or equal to 17 * 10¹²/L, the quantitative value is 0.006 * 10¹²/L; if the red blood cell parameter of the current sample is greater than 17 * 10¹²/L and less than or equal to 20 * 10¹²/L, the quantitative value is 0.008 * 10¹²/L; if the red blood cell parameter of the current sample is greater than 20 * 10¹²/L and less than or equal to 25 * 10¹²/L, the quantitative value is 0.010 * 10¹²/L; and if the red blood cell parameter of the current sample is greater than 25 * 10¹²/L, the quantitative value is 0.013 * 10¹²/L. It should be noted that specific quantitative values are related to the model and performance of the sample analysis apparatus itself, and each quantitative value may be obtained by means of actual measurement.

The sample analysis method in some embodiments provides a single-sample mode and/or a multi-sample mode. The single-sample mode, which means that the user manually puts a single sample into the sample analysis apparatus, is a means or mode of manual sample injection. The multi-sample mode, which means that the user puts a plurality of samples into the sample analysis apparatus in batches, is a means or mode of automatic sample injection. Considering that the number of body fluid samples is less than that of blood samples, in view of ease of sample injection, it is generally recommended that the user detects the body fluid samples by means of manual sample injection and detects the blood samples by means of automatic sample injection. In some embodiments, in the single-sample mode or in the manual sample injection mode, step 130 is performed for prompting. For example, the impact prompt for the same testing parameter as the sample currently tested of the next sample is generated according to the magnitude of at least one testing parameter of the current sample; or, the impact prompt for the same testing parameter of the sample currently to be determined in the single-sample mode is generated according to the magnitude of at least one testing parameter of a previous sample.

The sample analysis methods shown in FIGS. 14 and 15 may be combined; in some embodiments, the testing parameter involved in step 120 and the testing parameter involved in step 130 may be the same testing parameter, for example, both being the white blood cell count and/or the red blood cell count.

Descriptions are provided herein with reference to various exemplary embodiments. However, those skilled in the art should understand that changes and corrections may be made to the exemplary embodiments without departing from the scope of this specification. For example, various operational steps and assemblies for executing the operational steps may be implemented in different methods on the basis of specific applications or in consideration of any number of cost functions associated with operations of the system (for example, one or more steps may be deleted, modified or incorporated into other steps).

In addition, those skilled in the art can understand that the principles herein may be reflected in a computer program product in a computer-readable storage medium, where the readable storage medium is loaded with computer-readable program codes in advance. Any tangible non-transitory computer-readable storage medium may be used, including a magnetic storage device (a hard disk, a floppy disk, or the like), an optical storage device (CD-ROM, DVD, Blu-ray disc, or the like), a flash memory, and/or the like. These computer program instructions may be loaded on a general-purpose computer, a special-purpose computer, or other programmable data processing devices to form a machine, such that these instructions, when executed on a computer or other programmable data processing apparatuses, may produce a means for implementing a specified function. These computer program instructions may alternatively be stored in a computer-readable memory. The computer-readable memory may instruct a computer or other programmable data processing devices to operate in a particular manner, such that the instructions stored in the computer-readable memory may produce an article of manufacture which includes a means for implementing a specified function. The computer program instructions may alternatively be loaded onto a computer or other programmable data processing devices to perform a series of operational steps on the computer or other programmable devices to produce a computer-implemented process, such that the instructions, when executed on the computer or other programmable devices, may provide steps for implementing a specified function.

Although the principles herein are shown in various embodiments, many modifications of structures, arrangements, proportions, elements, materials, and components particularly applicable to specific environmental and operating requirements may be made without departing from the principles and scope of the disclosure. These modifications and other changes or corrections fall within the scope of this specification.

The foregoing detailed descriptions are provided with reference to various embodiments. However, those skilled in the art should understand that various corrections and changes may be made without departing from the scope of the disclosure. Therefore, the disclosure is intended for an illustrative purpose other than a limitative purpose, and all these modifications fall within the scope of the disclosure. Similarly, the advantages, other advantages, and solutions to problems of the various embodiments are described above. However, the benefits, the advantages, the solutions to the problems, and any of their contributing factors, or solutions clarifying them should not be construed to be critical, necessary, or essential. The term "include" used herein and any other variations thereof all refer to a non-exclusive inclusion, such that a process, method, article, or device including a list of elements includes not only these elements, but also other elements that are not expressly listed or not inherent to the process, method, system, article, or device. In addition, the term "couple" used herein and any other variations thereof refer to a physical connection, an electrical connection, a magnetic connection, an optical connection, a communication connection, a functional connection, and/or any other connections.

Those skilled in the art should understand that many changes may be made to the details of the foregoing embodiments without departing from the basic principles of the disclosure. Therefore, the scope of the disclosure should be determined in accordance with the following claims.

## Claims

1. A sample analysis apparatus, **characterized by** comprising:
a sample supply portion configured to supply a sample, the sample comprising a blood sample or a body fluid sample;
a reagent supply portion configured to supply a reagent;
a reaction portion configured to provide a reaction place for the sample and the reagent so as to prepare a test sample formed by reaction of the sample and the reagent;
a determination portion configured to test the test sample so as to obtain test data;
a processor that obtains one or more testing parameters of the sample with respect to particles according to the test data; and at least one testing parameter, the processor performing control according to the magnitude of the testing parameter to display the testing parameter with different precisions.

2. The sample analysis apparatus of claim 1, **characterized in that**, the processor performing control according to the magnitude of the testing parameter to display the testing parameter with different precisions comprises:
when the testing parameter is less than a threshold, the processor performs control to display the testing parameter with a first precision; conversely, the processor performs control to display the testing parameter with a second precision; where the first precision is higher than the second precision.

3. The sample analysis apparatus of claim 1 or 2, **characterized in that** the processor performs control according to the magnitude of a white blood cell count parameter to display the white blood cell count parameter with different precisions.

4. The sample analysis apparatus of claim 3, **characterized in that** the first precision is 0.001 * 10⁹/L, and the second precision is 0.01 * 10⁹/L.

5. The sample analysis apparatus of claim 1 or 2, **characterized in that** the processor performs control according to the magnitude of a red blood cell count parameter to display the red blood cell count parameter with different precisions.

6. The cell analysis apparatus of claim 5, **characterized in that** the first precision is 0.001 * 10¹²/L, and the second precision is 0.01 * 10¹²/L.

7. The sample analysis apparatus of claim 2, **characterized in that**, in response to a setting command, the processor sets the threshold.

8. The sample analysis apparatus of claim 1, **characterized in that** the processor further generates an impact prompt for the same testing parameter as the sample currently tested of a next sample according to the magnitude of at least one testing parameter of the sample currently tested.

9. The sample analysis apparatus of claim 8, **characterized in that** the impact prompt comprises a quantitative value to indicate that when an actual value of the same testing parameter as the sample currently tested of the next sample is less than the quantitative value, it is required to perform blank counting first when the next sample is tested, otherwise, a resulting test value of the testing parameter which is the same as the sample currently tested of the next sample is unreliable.

10. The sample analysis apparatus of claim 8 or 9, **characterized in that** the processor generates an impact prompt for a white blood cell count parameter of the next sample according to the magnitude of the white blood cell count parameter of the sample currently tested.

11. The sample analysis apparatus of claim 10, **characterized in that**, if the white blood cell parameter of the current sample is greater than or equal to 60 * 10⁹/L and less than or equal to 100 * 10⁹/L, the quantitative value is 0.003 * 10⁹/L; if the white blood cell parameter of the current sample is greater than 100 * 10⁹/L and less than or equal to 200 * 10⁹/L, the quantitative value is 0.008 * 10⁹/L; if the white blood cell parameter of the current sample is greater than 200 * 10⁹/L and less than or equal to 300 * 10⁹/L, the quantitative value is 0.010 * 10⁹/L; and if the white blood cell parameter of the current sample is greater than 300 * 10⁹/L, the quantitative value is 0.015 * 10⁹/L.

12. The sample analysis apparatus of claim 8 or 9, **characterized in that** the processor generates an impact prompt for a red blood cell count parameter of the next sample according to the magnitude of the red blood cell count parameter of the current sample.

13. The sample analysis apparatus of claim 12, **characterized in that**, if the red blood cell parameter of the current sample is greater than or equal to 12 * 10¹²/L and less than or equal to 17 * 10¹²/L, the quantitative value is 0.006 * 10¹²/L; if the red blood cell parameter of the current sample is greater than 17 * 10¹²/L and less than or equal to 20 * 10¹²/L, the quantitative value is 0.008 * 10¹²/L; if the red blood cell parameter of the current sample is greater than 20 * 10¹²/L and less than or equal to 25 * 10¹²/L, the quantitative value is 0.010 * 10¹²/L; and if the red blood cell parameter of the current sample is greater than 25 * 10¹²/L, the quantitative value is 0.013 * 10¹²/L.

14. The sample analysis apparatus of claim 8, **characterized in that**, the testing parameter for which the impact prompt is generated, and the testing parameter displayed with different precisions under the control are the same testing parameter.

15. The cell analysis apparatus of claim 1, **characterized in that** the body fluid sample comprises at least one of cerebrospinal fluid, pleural effusion, peritoneal effusion, pericardial effusion, and joint effusion.

16. A sample analysis apparatus, **characterized by** comprising:
a sample supply portion configured to supply a sample, the sample including a blood sample or a body fluid sample;
a reagent supply portion configured to supply a reagent;
a reaction portion configured to provide a reaction place for the sample and the reagent so as to prepare a test sample formed by reaction of the sample and the reagent;
a determination portion configured to test the test sample so as to obtain test data; and
a processor configured to obtain one or more testing parameters of the sample with respect to particles according to the test data, the processor further generating an impact prompt for the same testing parameter as the sample currently tested of a next sample according to the magnitude of at least one testing parameter of the sample currently tested.

17. The sample analysis apparatus of claim 16, **characterized in that** the impact prompt comprises a quantitative value to indicate that when an actual value of the same testing parameter as the sample currently tested of the next sample is less than the quantitative value, it is required to perform blank counting first when the next sample is tested, otherwise, a resulting test value of testing parameter which is the same as the sample currently tested of the next sample is unreliable.

18. The sample analysis apparatus of claim 16 or 17, **characterized in that** the processor generates an impact prompt for a white blood cell count parameter of the next sample according to the magnitude of the white blood cell count parameter of the sample currently tested,.

19. The sample analysis apparatus of claim 18, **characterized in that**, if the white blood cell parameter of the sample currently tested is greater than or equal to 60 * 10⁹/L and less than or equal to 100 * 10⁹/L, the quantitative value is 0.003 * 10⁹/L; if the white blood cell parameter of the sample currently tested is greater than 100 * 10⁹/L and less than or equal to 200 * 10⁹/L, the quantitative value is 0.008 * 10⁹/L; if the white blood cell parameter of the sample currently tested is greater than 200 * 10⁹/L and less than or equal to 300 * 10⁹/L, the quantitative value is 0.010 * 10⁹/L; and if the white blood cell parameter of the sample currently tested is greater than 300 * 10⁹/L, the quantitative value is 0.015 * 10⁹/L.

20. The sample analysis apparatus of claim 16 or 17, **characterized in that** the processor generates an impact prompt for a red blood cell count parameter of the next sample according to the magnitude of the red blood cell count parameter of the current sample.

21. The sample analysis apparatus of claim 20, **characterized in that**, if the red blood cell parameter of the current sample is greater than or equal to 12 * 10¹²/L and less than or equal to 17 * 10¹²/L, the quantitative value is 0.006 * 10¹²/L; if the red blood cell parameter of the current sample is greater than 17 * 10¹²/L and less than or equal to 20 * 10¹²/L, the quantitative value is 0.008 * 10¹²/L; if the red blood cell parameter of the current sample is greater than 20 * 10¹²/L and less than or equal to 25 * 10¹²/L, the quantitative value is 0.010 * 10¹²/L; and if the red blood cell parameter of the current sample is greater than 25 * 10¹²/L, the quantitative value is 0.013 * 10¹²/L.

22. The sample analysis apparatus of any one of claims 16 to 21, **characterized in that** the sample analysis apparatus further comprises a single-sample mode; and in the single-sample mode, the processor generates an impact prompt for the same testing parameter of the next sample according to the magnitude of at least one testing parameter of the current sample.

23. The sample analysis apparatus of claim 16, **characterized in that**, for at least one testing parameter, the processor performs control according to the magnitude of the testing parameter to display the testing parameter with different precisions.

24. The sample analysis apparatus of claim 23, **characterized in that**, the processor performing control according to the magnitude of the testing parameter to display the testing parameter with different precisions comprises:
when the testing parameter is less than a threshold, the processor performs control to display the testing parameter with a first precision; conversely, the processor performs control to display the testing parameter with a second precision; where the first precision is higher than the second precision.

25. The sample analysis apparatus of claim 23 or 24, **characterized in that** the processor performs control according to the magnitude of a white blood cell count parameter to display the white blood cell count parameter with different precisions.

26. The sample analysis apparatus of claim 25, **characterized in that** the first precision is 0.001 * 10⁹/L, and the second precision is 0.01 * 10⁹/L.

27. The sample analysis apparatus of claim 23 or 24, **characterized in that** the processor performs control according to the magnitude of a red blood cell count parameter to display the red blood cell count parameter with different precisions.

28. The sample analysis apparatus of claim 27, **characterized in that** the first precision is 0.001 * 10¹²/L, and the second precision is 0.01 * 10¹²/L.

29. The sample analysis apparatus of claim 24, **characterized in that**, in response to a setting command, the processor sets the threshold.

30. The sample analysis apparatus of claim 23, **characterized in that**, the testing parameter for which the impact prompt is generated, and the testing parameter displayed with different precisions under the control are the same testing parameter.

31. The sample analysis apparatus of claim 16, **characterized in that** the body fluid sample comprises at least one of cerebrospinal fluid, pleural effusion, peritoneal effusion, pericardial effusion, and joint effusion.

32. A sample analysis method, **characterized by** comprising:
preparing a test sample from a sample and a reagent, the sample being a blood sample or a body fluid sample;
testing the test sample to obtain one or more testing parameters of the sample with respect to particles; and
for at least one testing parameter, performing control according to the magnitude of the testing parameter to display the testing parameter with different precisions.

33. The sample analysis method of claim 32, **characterized in that**, performing control according to the magnitude of the testing parameter to display the testing parameter with different precisions comprises:
when the testing parameter is less than a threshold, performing control to display the testing parameter with a first precision; conversely, performing control to display the testing parameter with a second precision; where the first precision is higher than the second precision.

34. The sample analysis method of claim 32 or 33, **characterized in that**, performing control according to the magnitude of the testing parameter to display the testing parameter with different precisions comprises:
performing control according to the magnitude of a white blood cell count parameter to display the white blood cell count parameter with different precisions; and/or
performing control according to the magnitude of a red blood cell count parameter to display the red blood cell count parameter with different precisions.

35. The sample analysis method of claim 32, **characterized by** further comprising: generating an impact prompt for the same testing parameter of a next sample according to the magnitude of at least one testing parameter of the current sample.

36. The sample analysis method of claim 35, **characterized in that** the impact prompt comprises a quantitative value to indicate that when an actual value of the same testing parameter of the next sample is less than the quantitative value, it is required to perform blank counting first when the next sample is tested, otherwise, a resulting test value of the same testing parameter is unreliable.

37. The sample analysis method of claim 35, **characterized in that**, generating an impact prompt for the same testing parameter of a next sample according to the magnitude of at least one testing parameter of the current sample comprises:
generating an impact prompt for a white blood cell count parameter of the next sample according to the magnitude of the white blood cell count parameter of the current sample; and/or
generating an impact prompt for a red blood cell count parameter of the next sample according to the magnitude of the red blood cell count parameter of the current sample.

38. A sample analysis method, **characterized by** comprising:
preparing a test sample from a sample and a reagent, the sample being a blood sample or a body fluid sample;
testing the test sample to obtain one or more testing parameters of the sample with respect to cells; and
generating an impact prompt for the same testing parameter as the sample currently tested of a next sample according to the magnitude of at least one testing parameter of the sample currently tested.

39. The sample analysis method of claim 38, **characterized in that** the impact prompt comprises a quantitative value to indicate that when an actual value of the same testing parameter of the next sample is less than the quantitative value, it is required to perform blank counting first when the next sample is tested, otherwise, a resulting test value of the same testing parameter is unreliable.

40. The sample analysis method of claim 38, **characterized in that**, generating an impact prompt for the same testing parameter as the sample currently tested of a next sample according to the magnitude of at least one testing parameter of the sample comprises currently tested:
generating an impact prompt for a white blood cell count parameter of the next sample according to the magnitude of the white blood cell count parameter of the sample currently tested; and/or
generating an impact prompt for a red blood cell count parameter of the next sample according to the magnitude of the red blood cell count parameter of the sample currently tested.

41. The sample analysis method of claim 38, **characterized by** further comprising: for at least one testing parameter, performing control according to the magnitude of the testing parameter to display the testing parameter with different precisions.

42. The sample analysis method of claim 41, **characterized in that**, performing control according to the magnitude of the testing parameter to display the testing parameter with different precisions comprises:
when the testing parameter is less than a threshold, performing control to display the testing parameter with a first precision; conversely, performing control to display the testing parameter with a second precision; where the first precision is higher than the second precision.

43. The sample analysis method of claim 41 or 42, **characterized in that**, performing control according to the magnitude of the testing parameter to display the testing parameter with different precisions comprises:
performing control according to the magnitude of a white blood cell count parameter to display the white blood cell count parameter with different precisions; and/or
performing control according to the magnitude of a red blood cell count parameter to display the red blood cell count parameter with different precisions.
